# EUROPEAN PATENT APPLICATION

(11) **EP 2 060 637 A1**
(43) Date of publication of application: **20.05.2009**
(21) Application number: 07120703.9
(22) Date of filing: 14.11.2007
(51) Int. Cl.: C12Q 1/68

(54) **Means and methods for detection of nucleic acids**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention related to methods and tools for nuclease-dependent detection of nucleic acid hybridization. In these methods magnetic or magnetizable particles are used to discriminate between hybridized and non-hybridized DNA.

## Description

### FIELD OF THE INVENTION

The present invention relates to methods and devices such as biosensors for detecting nucleic acids, e.g. for use in molecular diagnostics.

### BACKGROUND OF THE INVENTION

The use of magnetic particles in nucleic acid detection methods is well known. W02006131892, for example, describes a magnetic biosensor wherein nucleic acids are amplified. During the amplification a magnetic particle is incorporated in the amplified DNA. The amplified nucleic acids are subsequently detected using the incorporated magnetic particles. The detection method involves the binding of a magnetic particle near a sensor surface. The binding near the sensor surface is achieved via the binding of the target to a probe attached to the sensor surface. In these methods, stringency steps must be added to obtain a high sensitivity and specificity for the probe/target interaction. This generally involves varying the temperature and/or buffer conditions.

The prior art suggests hybridization assays on a solid surface in which the signal generation is controlled by the action ofnucleases (WO03052139, W002059364). In these disclosures, methods are described wherein a nucleic acid target/probe hybrid is cleaved by an enzyme, resulting in the detachment of a detectable signal. Such enzymatic cleavage step renders the assay more selective and stringent. The possibility to choose enzymes which are selective for certain base sequences further increases specificity of detection. In some cases even single nucleotide mismatches (e.g. SNPs) can be detected. W002059364 suggests the use of magnetic particles as labels.

The assays of the prior art have several disadvantages that are a consequence of performing the detection near to or on the reaction surface. There is a need for a washing step after the enzymatic digestion to ensure low background signals and to eliminate false positives. Although it is possible to wait for the cleaved labels to diffuse away from the surface, this increases significantly the time-to-result. In applications involving rapid detection, a reduction in number of process steps is desirable. Secondly, because the labels are attached to a surface, the dynamic range of concentrations measurable can be limited compared to homogeneous assays where the signal is generated in the bulk volume.

Additionally, there are few methods which allow amplification of a signal generated from a target without amplifying the target itself or adding more labels. Such methods would be beneficial for the detection of low concentrations of nucleic acid target without amplification of this target.

Nucleic acids with nuclease activity (DNAzymes) have been used for the quantitative real-time detection of nucleic acids during amplification. In a homogenous phase, Qzyme probes (Clontech) employ DNAzymes to control the production of light through enzymatic cleavage of a tether between donor and acceptor dyes. Dyes have also been combined with gold nanoparticles as quenchers in probes. The detection of these probes is done optically using methods including fluorescence and colorimetric methods. These quantitative methods require a complex design of probe and dye labels.

### SUMMARY OF THE INVENTION

The present invention discloses methods for the detection of nucleic acid targets based on the manipulation of magnetic or magnetizable particles, which are released from a probe, towards a sensor surface. This release is the result of the action of a cleaving enzyme. These methods can be applied for instance in the quantitative detection of nucleic acids. They can optionally combined with amplification techniques (e.g. PCR).

Particular and preferred aspects of the invention are set out in the accompanying independent and dependent claims. Features from the dependent claims may be combined with features of the independent claims and with features of other dependent claims as appropriate and not merely as explicitly set out in the claims.

One aspect of the present invention relates to methods for detecting a target nucleic acid molecule (1) in a sample. Most particularly, the present invention relates to methods which combine the use of magnetic particles which are moved by a magnetic field and the action of nucleic acid specific enzymes. In particular embodiments, methods for detecting a target nucleic acid molecule (1) in a sample are provided which comprise the steps of:
a) providing a nucleic acid probe (2), complementary to the target nucleic acid molecule (1), wherein the probe (2) is linked to a magnetic or magnetizable particle (3) and is attached to a surface (4),
b) contacting the sample with the probe under conditions allowing the binding of the probe (2) to the target nucleic acid (1) thereby forming a probe/target hybrid,
c) applying or activating a nucleic acid cleaving enzyme (E), which discriminates between unbound probe and bound probe/target hybrid,
d) applying a magnetic field (F), such as to manipulate unbound magnetic or magnetizable particles towards a zone remote from the surface, at least during step (c),
e) detecting at one or more time points the concentration of magnetic or magnetizable particles on the surface (4) and/or the concentration of magnetic or magnetizable particles in the zone remote from the surface,

In particular embodiments, detection at the surface and/or in the zone away from the surface is performed by measuring the magnetic field of the magnetic or magnetizable particle.

In further embodiments, detection of magnetic or magnetizable particles at the surface and/or in the zone remote from the surface is performed by FTIR (frustrated total internal reflection).

In further particular embodiments, detection of magnetic or magnetizable particles at the surface and/or in the zone remote from the surface is performed by measuring a detectable label attached to the magnetic or magnetizable particle.

In particular embodiments, magnetic or magnetizable particles used in the context of the invention are particles comprising magnetic material and a fluorescent label.

In particular embodiments, methods of the invention make use of a nucleic acid cleaving enzyme which cleaves at least the probe in the probe/target hybrid.

In alternative embodiments, methods of the invention make use of a nucleic acid cleaving enzyme which cleaves double stranded DNA.

In particular embodiments, methods of the invention make use of a nucleic acid cleaving enzyme which is a sequence specific enzyme or is an enzyme specific for methylated nucleic acids.

In certain embodiments of methods described herein, a nicking enzyme is added prior to the nucleic acid cleaving enzyme.

In particular embodiments, methods of the invention make use of a nucleic acid cleaving enzyme which is a DNA- or RNAzyme which forms a part of the probe (2).

In yet further embodiments methods of the invention make use of a nucleic acid cleaving enzyme which is incorporated in the target nucleic acid molecule (1) during a PCR amplification of the target nucleic acid molecule (1).

In particular embodiments, methods of the invention make use of a nucleic acid cleaving enzyme which cleaves DNA/RNA hybrids.

In further embodiments, methods are described wherein the target nucleic acid (1) is DNA and the probe (2) is RNA and wherein the enzyme (E) is an enzyme that only digests RNA in an RNA/DNA hybrid.

A further aspect of the invention relates to devices for the detection of target nucleic acids based on the detection of magnetic or magnetizable particles. Devices according to the invention comprise a reactions surface (4) and a detection zone, wherein the detection zone is either a detection zone capable of detecting a label bound to the surface or a detection zone capable of detecting a label in a zone remote from the surface. Typically, devices described herein further comprise a means for moving magnetic or magnetizable particles away from and/or towards a detection zone capable of detecting a label bound to the surface and/or away from and/or towards or a detection zone capable of detecting a label in a zone remote from the surface. In further particular embodiments, devices of the invention comprise:
- a reaction surface (4),
- a first sensor (61) with a first detection zone capable of detecting a label bound to the reaction surface (4),
- a means for applying a magnetic field (F) from the detection surface to a zone remote from the detection surface
- a second sensor (62) with a second detection zone remote from the detection surface, and
- optionally, a means for moving magnetic or magnetizable particles towards the second detection zone.

In particular embodiments devices comprise a reaction chamber, wherein the reaction chamber comprises:
- a first region comprising the reaction surface (4) and the first detection zone capable of detecting a label bound to the reaction surface (4),
- a second region remote from the first region, comprising the second detection region capable of detecting a label in the region remote from the first region, and
- a means for applying a magnetic field (F) from the first region to the second region.

In a particular embodiments of devices described herein, the first and/or second sensors are capable of detecting different labels.

In further particular embodiments devices envisaged herein, further comprise a plurality of different probes with magnetic or magnetizable particles attached to the reaction surface, wherein each probe comprises a different detectable label.

In yet further particular embodiments, the first and second region of the devices envisaged herein are on opposite sides of the reaction chamber.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE FIGURES

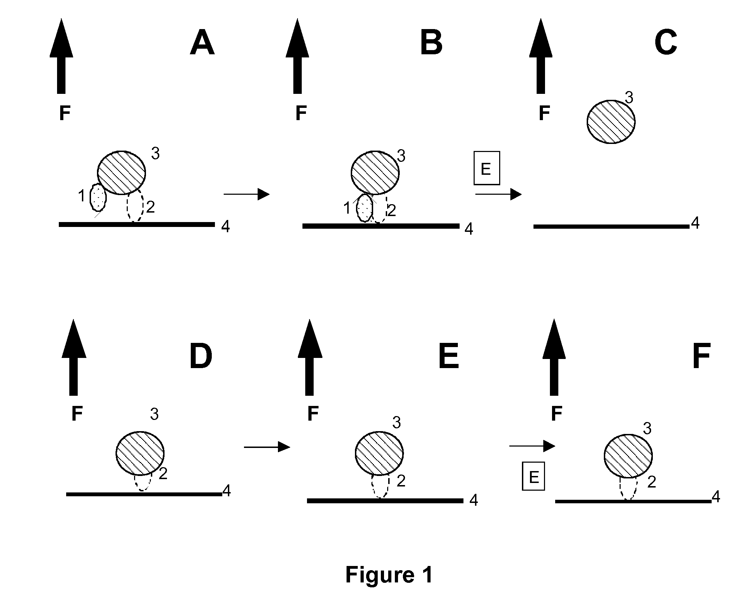

Figure 1 shows a schematic drawing of a particular embodiment of the nuclease dependent detection of nucleic acid probe/target hybridization according to the present invention.
   Panels A to C depict the situation wherein a target nucleic acid present in the sample binds to a probe (A: target nucleic acid present but not yet hybridized). The presence of a probe/target hybrid (B: formation of probe/target hybrid) results in the cleavage of this hybrid by an enzyme digesting only double stranded nucleic acids (Panel C) and in the release of a magnetic or magnetizable particle from the probe (C: released magnetic or magnetizable particle is moved away from reaction surface by magnetic field F).
   Panels D to F depict the fate of (excess) probe which did not hybridize with target (which also corresponds to the situation wherein no target nucleic acid is present in the sample). No hybrid is formed, no digestion takes place and the magnetic or magnetizable particle is not released.
   (1: target nucleic acid; 2: probe nucleic acid; 3: magnetic or magnetizable particle; 4: reaction surface; E: nuclease enzyme; F magnetic force).

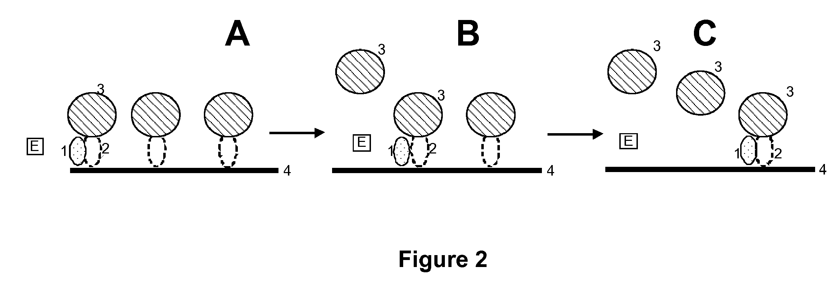

Figure 2 shows a schematic drawing of a particular embodiment of the present invention wherein signal amplification occurs without amplification of the target nucleic acid. In a first step (A), a nucleic acid target binds to a probe. An enzyme which digests only the probe in a probe/target hybrid is then added resulting in the release of the magnetic or magnetizable particle attached to the probe (panel B). The released target nucleic acid can then again bind to a probe (panel B). The probe in the probe/target hybrid is digested again resulting in the release of an additional magnetic or magnetizable particle (Panel C).
   1: target nucleic acid; 2: probe nucleic acid; 3: magnetic or magnetizable particle; 4: reaction surface; E: nuclease enzyme; F magnetic field.

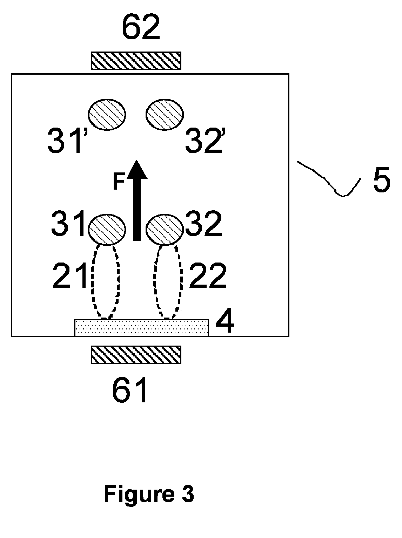

Figure 3 shows a schematic drawing of a device in accordance with an embodiment of the invention for performing a nuclease-dependent assay. The device comprises a reaction chamber (5) with a reaction surface (4) and two sensors (61 and 62) and a means for generating a magnetic field (F). Figure 3 shows an embodiment of the functioning of a device using different probes and labels for multiplexing. A fraction of each of the probes carrying magnetic or magnetizable particles and labels have been cleaved after hybridization and nuclease treatment. The magnetic or magnetizable particles (31) and (32) present on the fraction of undigested probes (21) and (22) remain in the proximity of sensor (61). Released magnetic or magnetizable particles (31') and (32') are manipulated toward sensor (62) by the magnetic force F.

In the different Figures, the same reference signs refer to the same or analogous elements.

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### DETAILED DESCRIPTION OF THE INVENTION

### Definitions

The term 'target' as used herein, refers to a nucleic acid to be detected and/or quantified in the methods of the present invention. A non-limiting list of examples of target molecules envisaged by the present application is provided in the description. The term target can refer to a nucleic acid as present in a sample but may also refer to an amplified version thereof (via PCR, AMR, TMR, NASBA...)).

The term 'probe' as used in the application refers to a nucleic acid comprising a sequence capable of specifically binding to (part of) a target.

'Nucleic acid' in the present invention refers to DNA and RNA as occurring in natural samples but also refers to synthetic molecules such as PNA (nucleic acids with a peptide backbone).

Magnetic beads, spheres or other shapes capable of being magnetized in a magnetic field are referred to as 'particles' when they are mentioned in the context of manipulation in a magnetic field.

The term 'label' as used herein refers to any compound which generates a detectable signal. In this context magnetic or magnetizable particles are referred to as 'labels' when they are mentioned in the context of detection of e.g. their magnetic field or of their optical properties.

The term 'reaction surface' refers to a surface to which a probe can be attached and where, in methods of the present invention, the nuclease digestion takes place.

A 'detection zone' (or region) as used herein, refers to a zone, optionally corresponding to a part of the reaction chamber, which is in the range of a sensor, allowing detection of a label within this zone. Where detection is performed on a surface, this is also referred to as a detection surface. The detection surface can be identical to or different from the reaction surface.

One aspect of the present invention relates to methods for detecting a target nucleic acid molecule in a sample. These methods are based on the binding of a target nucleic acid molecule to a probe, which, further to nuclease activity ensures the release of a detectable label. According to a particular embodiment, these methods comprise the following steps:
providing a nucleic acid probe (2) comprising a sequence complementary to the target nucleic acid molecule (1), wherein the probe (2) is linked to a magnetic or magnetizable particle (3) and is attached to a surface (4),
contacting a sample, suspected of containing the target nucleic acid, with the probe under conditions allowing the binding of the probe (2) to the target nucleic acid (1) thereby allowing, if the target nucleic acid is present in the sample, the formation of a probe/target hybrid,
applying or activating a nucleic acid cleaving enzyme (E), which discriminates between unbound probe and bound probe/target hybrid,
applying a magnetic field (F), so as to manipulate unbound magnetic or magnetizable particles towards a zone away from the surface,
detecting at one or more time points the number of magnetic or magnetizable particles on the surface (4) and/or the number of magnetic or magnetizable particles in a detection zone remote from the surface.

In the context of the present invention reference to a zone 'away from' or 'remote from the surface', refers to a zone characterized in that the presence of magnetic/magnetizable particles in that zone does not affect detection of magnetic particles at the surface and/or detection in that zone is not affected by the presence of magnetic/magnetizable particles on the surface. With regard to detection, the zone remote from the surface is independent from the zone at the surface.

Depending on the characteristics of the nuclease used, the detection of magnetic or magnetizable particles at the surface or in the zone away from the surface is either directly or inversely correlated with the amount of target nucleic acid in the sample.

In particular embodiments, the nuclease which discriminates between unbound probe and bound probe/target hybrid is capable of cleaving at least the probe in a probe/target hybrid and does not cleave unbound probe. In these embodiments, only the unbound probe will remain on the reaction surface. The signal generated at the reaction surface by the label on the unbound probe is inversely correlated with the amount of target in the sample. The signal generated by the released particles in a detection zone away from the binding surface (cleaved probe/target hybrids) is directly correlated with the amount of target in the sample.

In alternative embodiments the nuclease which discriminates between unbound probe and bound probe/target hybrid cleaves only unbound probe nucleic acid. In these embodiments, only probe/target hybrid formed upon contacting the probe with the sample remains on the reaction surface. The signal generated at the reaction surface by the label on the probe/target hybrid is directly correlated with the amount of target in the sample. The signal generated by the released particles in a zone remote from the binding surface (cleaved unbound probe) is inversely correlated with the amount of target in the sample.

In the methods envisaged herein, nucleic acid hybridization , nuclease cleaving and magnetic separation are combined. In particular embodiments of these methods neither the hybridization nor the nuclease digestion is influenced by the presence of the magnetic field. Accordingly, the application of the magnetic field need not be limited to those steps of the methods wherein the released magnetic or magnetizable particles are manipulated towards the zone away from the surface and/or wherein the presence of magnetic or magnetizable particles on the surface is detected. This has the advantage that detection can occur throughout the method and that detection of the target can be carried out simultaneously with e.g. PCR amplification of the target.

Methods of the present invention are applicable for the detection of nucleic acids in a wide variety of samples. Samples can be of any origin and include environmental samples, forensic samples, samples from industrial processes, microbial samples, tissue or body fluid samples, etc.. Methods of the present invention are applicable to DNA as well as RNA. In particular embodiments, the nucleic acids in a sample are processed prior to the assay, by e.g. removing proteins, separating DNA from RNA, isolating mRNA, converting RNA into DNA via reverse transcriptase, fragmentation of DNA via sonication or limited digestion, etc.

In particular embodiments DNA or RNA target nucleic acids are amplified prior or during the assays described herein via PCR or other methods such as SDA (Strand Displacement Amplification), TMA (Transcription Mediated Amplification) or NASBA (Nucleic Acid Sequence Based Amplification).

The nucleic acid probes which are used in methods of the present invention can be DNA, RNA or even synthetic nucleic acid like molecules such as PNA. Probes are typically synthesized via chemical methods, but can also be made by the isolation of nucleic acids from plasmids or other vectors. Alternatively, the probes are made by *in vitro* transcription and/or translation methods or by amplification methods such as PCR. In particular embodiments a probe optionally contains, in addition to the sequence capable of specifically binding to (part of) a target, a spacer. The function of such a spacer is to further increase the physical distance between the enzymatic cleavage site and the magnetic particle and/or between the enzymatic cleavage site and the reaction surface. In this way the enzyme is less hindered by the various surfaces. The spacer can be any species, nucleic acid, protein, small molecule, peptide etc. which is not a substrate for the nuclease used in the assay.

Within methods of the invention, a nucleic acid probe is attached to a surface via methods which are well known in the field of micro arrays. The surface is used as reaction surface and is typically part of a reaction chamber.

Different methods are known in the prior art for reacting nucleic acids with other compounds such as chemical coupling through reactive functional groups (-COOH, NH2, HS, epoxy, aldehyde, tosyl, maleimide etc) or coupling via intermediate binding groups and binding group pairs such as avidin/biotin, antibody/antigen, antibody/hapten, protein/protein, protein/lectin, protein/DNA, DNA/DNA, protein/aptamer. These and other methods can be used for attaching a probe to a surface as envisaged in methods of the invention.

Probes as used in methods described herein are typically further functionalized with a magnetic or magnetizable particle. Methods to bind magnetic or magnetizable particles to DNA are well known to the skilled person and are commercialized by e.g. Dynal.

For an optimal sensitivity, methods of the invention make use of magnetic particles for which the magnetic particle/probe ratio is 1.

In particular embodiments of methods envisaged herein, the magnetic or magnetizable particle functions as a label and is detected by a sensor capable of detecting the presence of the magnetic or magnetizable particle in its detection zone (e.g. on a detection surface). This can be ensured e.g. by detection of the magnetic field of the particles. Alternatively the magnetic particles are detected by their optical properties using e.g. FTIR.

In further particular embodiments of methods envisaged in the context of the present invention, probes are used which comprise, in addition to the magnetic or magnetizable particle, one or more other functional groups. Such groups are typically detectable labels such as optical labels (fluorescent, phosphorescent, SERRS) or ultrasound labels. These labels can be attached to the nucleic acid probe directly. In further particular embodiments, the magnetic or magnetizable particles comprise one or more functional groups, which can be used as a label. Labels can be attached via chemical methods to the surface of a magnetic or magnetizable particle or can be incorporated into the magnetic or magnetizable particle (e.g. fluorescent polymer particles containing paramagnetic material). The main requirement for methods described herein is that during the assay, e.g. as a result of nuclease treatment, the magnetic or magnetizable particle and detectable label are not separated from each other.

Methods of the present invention envisage the formation of DNA/DNA, DNA/RNA or RNA/RNA probe/target hybrids. Typically the sequence within the probe capable of specifically hybridizing with the target corresponds to an oligonucleotide which is shorter than the target nucleic acid. Optionally, as described above, probes may comprise a spacer. Factors such as the nature of the hybrid (DNA/DNA or DNA/RNA), the length of the probe/target hybrid, the degree of complementarity between probe and target have an influence on the hybridization. An appropriate choice of the length and the sequence of the probe, buffer composition and temperature allow a manipulation of the specific binding between probe and target. The selection of appropriate conditions is known to the skilled person and is explained for example in Sambrook et al. (2001) "Molecular Cloning: A Laboratory Manual", Cold Spring Harbor Press).

As detailed above, methods according to the invention encompass the cleavage of the probe (and optionally also the target) within the formed target/probe hybrid, or the cleavage of only single strand probe, i.e. not hybridized with the target, thereby ensuring release of the magnetic or magnetizable particle. This is typically ensured by adding or activating a nuclease (nucleic acid cleaving enzyme) to the target/probe hybrid on the reaction surface. The use of different types of nucleic acid cleaving enzymes is envisioned in methods of the present invention. The enzyme used can be any type of catalytic molecule that cleaves or digests nucleic acids including proteins, peptides and nucleozymes (DNAzymes, RNAzymes or a combination of DNA and RNA). The enzyme of choice can cleave at a single position or at multiple locations within the probe (including within the spacer therein). The choice of the appropriate enzyme depends on the nature and sequence of the probe and the target.

In particular embodiments of the invention, methods are provided which encompass the digestion of the probe/target hybrid. Herein, a nuclease is used which cleaves at least the probe, and optionally also the target, in the probe/target hybrid.

In particular embodiments, both target and probe are DNA. In such methods the enzyme of choice typically only acts on double stranded DNA. Examples hereof are double-strand specific DNAses such as T7 exonuclease and Exonuclease III.

The cleavage of double stranded DNA can also be performed with restriction endonucleases which recognize specific bases and cleave only at specific locations. These specific cleaving sequences can either be inherent in the target or a consequence of modification during a target amplification process. The incorporation of rare restriction enzyme recognition sites (e.g. 8bp cutters) significantly increases the specificity of the assay. In those assays wherein no amplification steps take place, the specificity can be increased by screening the sequence of target nucleic acid for a restriction enzyme recognition site which occurs less frequently and using this region for the design of a probe.

In other embodiments of methods described herein, both target and probe are RNA. In such methods the enzyme of choice typically only acts on double stranded RNA. An example hereof is RNAse III of *E. coli.*

In further embodiments of methods described herein, the target is DNA and the probe is RNA. In such methods the enzyme of choice typically only acts on the DNA/RNA hybrid wherein at least the RNA is cleaved. An example hereof is Rnase H, which specifically degrades RNA in a RNA/DNA hybrid, or T7 exonuclease, which degrades RNA and DNA in RNA/DNA hybrids, but does not degrade single stranded RNA.

In yet other embodiments of methods described herein, the target is RNA and the probe is DNA. In these methods the enzyme of choice typically only acts on the DNA/RNA hybrid wherein at least the DNA is cleaved such as Duplex specific nucleases (DSN) from kamchatka crab (Shagin et al. (2002) Genome Res. 12, 1935-1942).

In further particular embodiments, both target and probe are DNA wherein a mismatch (mutation or loop) occurs between the target and probe DNA. Enzymes such as CEL1 (US658322) or SP1 (Pimkin et al. (2007) BMC Biotechnol. 7, 29) can nick the DNA at the presence of the mismatch. Nicking enzymes are generally specific for double stranded nucleic acids and do not cleave single stranded nucleic acids. Optionally, exonucleases such as Nuclease Bal-31 or T7 Exonuclease can further degrade the nicked DNA and promote the release of magnetic or magnetizable particles.

In particular embodiments of the invention, methods are provided which encompass the digestion of only the unbound probe, i.e. probe which is not hybridized with the target. Herein, a nuclease is used which cleaves single stranded probe but which does not cleave the probe or the target in a probe/target hybrid.

Examples of enzymes suited for use in these methods include single-strand specific DNAses (e.g. Exonuclease 1, Mung Bean nuclease, Nuclease Ba131, RecJ_{f}, S1 nuclease) where DNA probes are used. In other embodiments the enzyme used is a single-strand specific RNAse (RNAse I, RNAse A, RNAse T1, Mung Bean nuclease, Nuclease Ba131) for RNA probes. The use of such enzymes is particularly suitable in assays wherein DNA/RNA hybrids are formed. In order to avoid undesired digestion of the probe prior to the binding of target, the single strand specific nuclease is added after the formation of target/probe hybrids.

In particular embodiments of the invention, the nuclease enzyme is a nucleic acid with catalytic properties. Most particularly, the nucleic acid enzyme is present within the probe and/or amplified target nucleic acid. In these embodiments, the enzyme does not need to be added to the probe, but rather is activated to ensure the required activity.

In a particular embodiment, a nucleic acid based enzyme (e.g. DNAzymes, and RNAzymes), is used whereby the catalytic properties are built into the probe making the DNA-or RNAzyme an integral part of the probe. An example of one such probe is the TASC (Target-Assisted-SelfCleaving) probe described in Sando et al. (2005 Org. Biomol. Chem. 3, 1002-1007). Binding of such a probe to the target results in a conformational change that causes the DNAzyme to cleave itself. A magnetic or magnetizable particle attached to a probe comprising such DNAzyme, can thus be cleaved off by the binding of the target to the probe and released.

In another embodiment, the probe contains, in addition to a sequence capable of hybridizing to the target, a sequence which is a substrate for the cleavage reaction of a nucleozyme. In this case the probe may have to fulfill 1 certain enzyme requirements such as the presence of specific ribonucleotide at the cleavage site. The relevant active nucleozyme is provided in the amplified nucleic acid target (e.g. via the primer in PCR) or released during amplification. Upon hybridization of the probe with the target, the magnetic or magnetizable particle is released. The amount of cleaved particle can be related to the concentration of nucleozyme and thus the concentration of target. Examples of nucleozymes include (Qzyme (Clontech), Hammerhead. The use of such nucleozymes allows the real time, quantitative detection of PCR products.

The nucleases envisaged in methods of the present invention may require particular conditions for their activity such as the presence of ATP, GTP etc and any cofactors (e.g. NAD+). These conditions are known and can be ensured at the reaction surface (e.g. by the use of suitable buffers).

Where applicable to the nuclease enzymes described above, the probe structure can be adapted to fulfill 1 the requirement of the appropriate nuclease, (e.g. a free 3' or 5' end, nick, gap). Furthermore if the target is to be subjected to a target amplification process (PCR, NASBA, SDA...) the amplified target can be modified (e.g. via the primers) to fulfill the requirements of the nuclease or to allow for similar adaptations in both target and probe.

Methods of the present invention encompass binding a magnetic or magnetizable particle to a surface through a probe and then releasing the magnetic or magnetizable particle and ensuring its movement away from the surface to which it was bound. More particularly methods described herein comprise a step wherein magnetic or magnetizable particles are manipulated by a magnetic field away from the reaction surface.

Methods of the present invention disclose quantitative assays for nucleic acid targets based on the binding of magnetic or magnetizable particles to a surface, specific activity of a cleaving enzyme releasing the particles and subjection of the magnetic or magnetizable particles to a magnetic force away from the surface, reducing the need for washing. In embodiments of methods described herein where the nuclease acts only on the probe/hybrid, applying a magnetic field pulling the particles away from the reaction surface when the nuclease is active further ensures that the cleaved magnetic or magnetizable particles are rapidly removed from the reaction surface without affecting amplification allowing real-time detection.

The signal generated by the presence of the target in the sample can be detected at two levels. First, the amount of particles bound to the reaction surface can be detected before and after the activity of the nuclease. In this embodiment the reaction surface functions as a detection zone, optionally as a detection surface. In those embodiments where cleavage of the label from probe/target hybrids only is envisaged, and labels can only be released from the surface in the presence of a target molecule, the number of particles released from the surface (and thus the decreasing signal on the reaction surface) is correlated to the amount of target present in the sample. In the embodiments wherein the nuclease is specific for single stranded probe, labels can only be released from those probes on the surface which do not bind to a target molecule. Thus in these embodiments, the number of particles released from the surface (and the decreasing signal on the reaction surface), is inversely correlated to the amount of target present in the sample

Secondly, the released magnetic or magnetizable particles can be measured in a detection zone (e.g. at a sensor surface) remote from the reaction surface, whereby particles released from the reaction surface are (at least in part) moved towards this detection zone (referred to herein as the second detection zone) by the magnetic field. This detection zone is remote from the reaction surface. Again, in those methods where only probe/target hybrids are cleaved, the number of particles detected in this detection zone remote from the reaction surface is directly correlated to the amount of target present in the sample (the more target present in the sample, the stronger the signal), while in methods which make use of nucleases degrading only single stranded probes, the signal detected in the second detection zone is inversely correlated with the amount of target in the sample (the more target present in the sample, the weaker the signal). Irrespective of whether both detection zones are used in different embodiments of methods described herein, the sensor and corresponding detection zone capable of detecting the presence of a label on the reaction surface is referred to as the first sensor/first detection zone, while the sensor and corresponding detection zone capable of detecting a label in a region remote from the reaction surface is referred to as the second sensor/second detection zone.

The possibility to perform two independent measurements enables the kinetic determination of the concentration of nucleic acid targets with high precision and within a large dynamic range, as several measurements can be made for one concentration. The use of magnetic or magnetizable particles which are removed from the reaction surface with a magnetic field further provides the option of physically collecting the particles and counting their number (e.g. in addition to or as a particular embodiment of the second sensor remote from the reaction surface). This allows the determination of the target concentration with high accuracy and precision.
It is noted that, in the detection schemes envisaged above, the detection both at the reaction surface and in a detection zone away from the reaction surface can occur either based on the magnetic or optical properties of the magnetic or magnetizable particles in the detection zone(s) or based on another label (other than the magnetic field) in or on the magnetic or magnetizable particle as detailed below, which is detectable in one or both detection zones.

Thus, in particular embodiments the magnetic properties of the magnetic or magnetizable particles are used themselves as labels for the detection. This can be done with any suitable electromagnetic sensor that can detect the presence of magnetic or magnetizable particles, e.g. an electrode, a wire, a coil, magneto-resistive sensor GMR, AMR TMR), magneto-strictive sensor, Hall sensor, planar Hall sensor, SQUID, magnetic resonance sensor. Alternatively, the detection occurs based on the optical properties of the magnetic or magnetizable particles themselves (e.g. FTIR detecting e.g. iron oxide).

Alternatively, or in combination with magnetic and/or FTIR detection, other labels or detection methods can be used. For example, magnetic or magnetizable particles can also be used for electrical detection. Alternatively, magnetic or magnetizable particles can comprise a mixture of a magnetic material and, for example, a fluorescent material, also allowing the detection of such particles with two independent methods. In other embodiments, a nucleic acid can be labeled for example separately with both magnetic or magnetizable particles and fluorescent labels (see above).

Methods wherein the magnetic or magnetizable particles are detected based on a material witch is not comprised within or attached to the magnetic or magnetizable particle itself are also envisaged. For instance, the probe to which the magnetic or magnetizable particle is bound can further comprise an optical label. Such embodiments of the methods of detection envisaged are only suitable in assays wherein the nucleic acid probe is not completely digested in the enzymatic digestion step (which would result in a release of the magnetic or magnetizable particle from the label). For instance, where the probe comprises a linker and the linker, the label may be linked to the linker, provided that at least this section of the linker to which the label is bound (and the region linking this section to the particle) is not subject to enzymatic cleavage. Detection methods which are based on the properties of the magnetic or magnetizable particles themselves, or of a material comprised within the particle or attached directly to the particle are particularly suitable for assays wherein the nucleic acid probe is completely digested and thus completely removed from the particle by the nuclease.

Where labels other than the magnetic or optical properties of the magnetic or magnetizable particles themselves are used, differential labeling of probes provides the possibility of performing multiplexing assays. For instance, different probes (i.e. binding different targets) are each labeled with a different label, such that the presence of different targets can be detected simultaneously. For example, the detection of different strains of a bacterium is envisaged. This can be performed in an assay wherein strain-specific probes bound to the reaction surface are each labeled with a polymer particle comprising magnetic material and a different fluorescent material. Depending on the type of strain present within the sample, a specific colored magnetic or magnetizable particle will be released from the surface after digestion of probe/target hybrids. As detailed above this can optionally be detected both at the reaction surface and/or in a detection zone remote from the reaction surface.

Those embodiments of methods described herein which rely on the selective cleavage of the probe in the probe/target hybrid only provide the additional advantage that the detection of the amount of label in either detection zone (or attached to either sensor surface) can proceed during the assay and that a measurement of the signal as a function of time can be used to determine the target concentration. Although the signal at a single time point is sufficient to evaluate the concentration, measurements at several time points are typically performed as this improves the precision. Another advantage of kinetic based measurements is that concentrations within a large range can be measured. For high concentrations, it may be desirable to use short reaction times as full removal of the labels from the reaction surface will be rapidly attained, whereas for low concentrations the amount of labels bound to the reaction surface is not limiting and long reaction times are desirable to achieve as large a signal change as possible. Assays that rely on endpoint or single time point measurements such as described in the prior art detect more limited concentration ranges. Kinetic detection of signals from high target concentrations can be measured in the short time regime (before all labels are removed from the reaction surface) and extrapolated to the long time regime or vice versa, signals from low target concentrations can be measured in the long time regime and interpolated to the short time regime. As the measurement can be completed in real-time, it is possible to determine, *in situ,* from the rate of reactions, whether a concentration falls in the high or low concentration regime.

In the measurement of signal change, a small change (i.e. low target concentration) in a large signal will have high error. In order to increase the precision of measurements, in particular embodiments of methods described herein, the number of labels released from the reaction surface is measured rather than the number of labels remaining attached to the surface. The magnetic force used to remove particles that have been released by the enzymatic cleaving reaction in combination with other force fields (which are preferably magnetic) are used to collect and redirect cleaved particles to another area where optionally their label is detected and their amount is determined. A measurement of both the cleaved and uncleaved particles results in very high precision in the determination of the target concentration. A particular configuration to allow simultaneous real-time measurement of cleaved and uncleaved particles is a reaction with a first sensor at the reaction surface, a second sensor in a detection zone (e.g. at a sensor surface) remote from but parallel to this reaction surface and a magnetic force capable of pulling particles away from the reaction surface and towards the second detection zone All cleaved particles are pulled away from the reaction surface and optionally held in place at the second detection zone by the magnetic force and are detected. Other possible configurations include adjacent sensors (e.g. two zones remote from each other in the same plane) in combination with a magnetic force which is capable of moving magnetic or magnetizable particles parallel to these surfaces from the first detection zone at the reaction surface to the second detection zone remote from the reaction surface. Further configurations include combinations of the magnetic field with e.g. microfluidic movement of the particles whereby after the removal from the reaction surface by the magnetic force, the particles are transported by microfluidics to a second detection zone.

Methods of the present invention allow the real-time detection of amplified target nucleic acids. In many applications including pathogen identification, viral load testing, food testing and mRNA expression profiling, the low quantity of target nucleic acid renders it necessary to amplify the target using various exponential (e.g. PCR, NASBA) and linear (e.g. LCR, TMA,) amplification techniques. The quantitative detection of these nucleic acid targets is often desirable, e.g. in certain diagnostic methods. The real-time detection of target nucleic acids during amplification enables the coupling of the rate of copy formation to the original target nucleic acid concentration. Accordingly, methods of the present invention further enable determining in real-time the rate of amplification of nucleic acid targets which act as templates. In these embodiments, the nucleic acid probe contains a segment that can be cleaved by the action of a nucleozyme (DNA, RNA or a combination). As described above, the activation of the nucleozyme can occur during the amplification of the nucleic acid target. More specifically, the cyclic process of template duplication (polymerase binding, primer binding, or strand extension) can result in the release, activation or production of nucleozymes. In the case of PCR and NASBA, it is possible to use for example a primer containing a template for the nucleozyme such that strand extension results in the generation of an active nucleozyme sequence attached to the amplicons (such a primer is the Qzyme primer disclosed in W02007056312A2). The active nucleozyme can hybridise with the nucleic acid probe containing the necessary recognition sequence and release magnetic or magnetizable particles (i.e. labels) from the reaction surface. With each round of amplification, the number of enzymes increases in direct proportion to the number of amplicons. The exponential increase in enzyme activity is observed in the increase in released labels. As in standard real-time PCR, the higher the concentration of target, the fewer the number of cycles required to reach a certain detection level. As free labels can only occur in solution due to enzyme action, the background signals will be very low.

In particular embodiments, methods of the present invention are used for the detection of methylated target nucleic acids. DNA methylation generally occurs at CpG sites (cystosine followed by guanine in sequence) by DNA methylating enzymes. In bacteria, methylation occurs at adenine sites by enzymes such as by EcoR I methylase (recognizes GAATTC sequences). Also RNA methylases are described which methylate RNA substrates. Depending on the design of the assay (methylated or unmethylated probe DNA) certain endonucleases can be used for which the cleavage is dependent on the presence of methylated DNA (e.g. MCRBc, DpnI) or the absence of methylated DNA (e.g. DpnII, SacI). These assays are suitable e.g. for tumour diagnosis since the methylation state of a number of genes is indicative of certain cancers.

Another aspect of the present invention provides devices for performing methods such as those described herein. More particularly devices are provided which allow the movement of magnetic or magnetizable particles from the reaction surface to a zone remote from the reaction surface and a sensor with a detection zone capable of detecting particles either at the reaction surface and/or remote from the reaction surface. In particular embodiments, devices according to the invention comprise:
a reaction surface (4)
a detection zone remote from the reaction surface
a means for applying a magnetic field capable of moving magnetic or magnetizable particles from the reaction surface to a zone remote from the reaction surface.

Particular embodiments of devices as envisaged herein further comprise a sensor (referred to herein as the first sensor) ensuring a first detection zone capable of detecting a label bound to the reaction surface.

In further particular embodiments, the means for applying a magnetic field ensures the presence of a magnetic field between the reaction surface and a detection zone remote from the reaction surface. Alternatively, devices are envisaged which further comprise, in addition to the means for applying the magnetic field, a means for moving magnetic or magnetizable particles towards the second detection zone.

In devices comprising both a first and a second detection zone, the first and second detection zones can be positioned at different places in the device, as long as the detection of a label in one detection zone is not influenced by the presence of a label in another detection zone. Typical arrangements are the positioning of the detection zones at opposite sides of the reaction chamber or next to (i.e. in the same plane) but sufficiently remote from each other.

In alternative embodiments, devices are provided comprising only the hereinabove described second detection zone, which is remote from the reaction surface.

In particular embodiments, devices are provided comprising a reaction chamber, wherein the reaction chamber comprises: a first region comprising a reaction surface and a sensor ensuring a first detection zone capable of detecting a label bound to the reaction surface and a second region remote from the first region, comprising a second sensor (and corresponding detection zone) capable of detecting a label in a region remote from the first region. Such devices further comprises a means for applying a magnetic field between the first region and the second region so as to ensure the movement of particles from/to the surface and/or from/to the detection zone remote from the surface.

In further particular embodiments devices are provided which comprise:
- a reaction chamber comprising:
   a first region of the reaction chamber comprising a reaction surface (4) and a sensor (61) ensuring a first detection zone in the proximity of the reaction surface for detecting magnetic or magnetizable particles (3) bound to the reaction surface
   a second region of the reaction chamber remote from the first region, comprising a second sensor (62) ensuring a second detection zone for detecting magnetic or magnetizable particles released from the reaction surface, and
- a means for applying a magnetic field (F) from the first region to the second region.

As detailed above the first and/or second sensor can be the same or different sensors which detect the magnetic, optical or electric properties of a magnetic or magnetizable particle and/or sensors which detect other labels present directly on the magnetic or magnetizable particle, within the magnetic or magnetizable particle or indirectly bound to the magnetic or magnetizable particle through part of the probe.

In particular embodiments, devices can be provided ready for use, i.e. comprising suitable nucleic acid probes (2) attached to the reaction surface whereby the probes carry a magnetic or magnetizable particle.

In particular embodiments, a sensor is provided with a detection zone capable of detecting labels bound to the reaction surface. In further particular embodiments the reaction surface can be in a transparent material such as glass or plastic (e.g. polystyrene or polycarbonate), which allows the detection of optic (e.g. fluorescent) labels bound to the probes attached to the glass reaction surface.

In particular embodiments, the sensor capable of detecting a label bound to the reaction surface specifically detects only labels present in a region in the close proximity of or on the reaction surface, rather than detecting the bulk solution present in a region near to the reaction surface. As indicated above, the sensor can be any sensor suitable for detecting the presence of magnetic labeled nucleic acids particles on the reaction surface, based on any property of the magnetic or magnetizable particles themselves, e.g. it can detect via magnetic methods (e.g. magnetoresistive, Hall, coils), optical methods such as FTIR (frustrated total internal reflection), sonic detection (surface acoustic wave, bulk acoustic wave, cantilever, quartz crystal etc), or electrical detection (e.g. conduction, impedance, amperometric, redox cycling). In particular embodiments the magnetic particles are detected by FTIR. Herein, the sensor surface is made of a material with a high transparency for light of a given spectral range (e.g. glass or some transparent plastic). The refractive index of this material is larger than the refractive index of the sample adjacent to the sensor surface. The sensor surface is illuminated with a light beam (from e.g. a laser or LED) under an angle such that it is totally internally reflected at the interface between the sensor surface and the sample. Under control conditions all light will be reflected at this interface. Only when particles are bound near the interface (e.g. high-index polystyrene with magnetic grains) the total internal reflection is frustrated, by absorption or scattering of light and a drop in reflected intensity can be measured.

Additionally or alternatively the sensors are sensors suitable for detecting the labels attached directly or indirectly thereto, e.g. via imaging, fluorescence, chemiluminescence, absorption, scattering, surface plasmon resonance, Raman, etc. or one of the methods described above.

In particular embodiments the first and or second sensor are capable of detecting more than one label and/or more than one type of label. This is of interest in the context of performing multiplexing in the methods described herein.

In further particular embodiments the reaction surface and corresponding first detection zone are gridded allowing the differential detection of signals in different regions on the reaction surface. This also allows multiplexing in methods of the invention. Methods for performing multiplexing are detailed hereafter.

Multiplexing using methods of the present invention is performed by using different probes (i.e. directed to different targets) attached to the reaction surface. These probes can be either identically labeled (e.g. the label is the presence of an identical magnetic or magnetizable particle on all probes) or differentially labeled.

In a first embodiment, the different probes are identically labeled (different probes are for instance applied on separate regions (e.g. within a grid) of the reaction surface, which can be distinguished within the detection zone by the sensor. The decrease in signal intensity is measured simultaneously (or consecutively) for each region within the grid.

A more elegant way of multiplexing uses different probes which each have a different detectable label. An example hereof are probes which carry beads comprising magnetic material and different fluorescent labels which can be discriminated from each other. In this set-up the different probes can be attached arbitrarily to the reaction surface and, upon release of the particles from the surface, the decrease in label at the detection surface and/or the increase in label in a second detection zone remote from the reaction surface can be detected, whereby the magnetic or magnetizable particles originating from different probes are distinguished based on the different fluorescent label.

The qualitative and/or quantitative determination targets in various bodily samples has applications in e.g. the diagnosis of various diseases such as genetic diseases or infectious disorders.

For routine analysis the reagents can be provided as ready to use reaction chambers or parts of reaction chambers, wherein probes, enzymes and buffers are stored as dried or frozen components.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1 : Nuclease dependent detection of probe/target binding

The present example illustrates an embodiment of the present invention. A nucleic acid probe (2) is labeled with a magnetic or magnetizable particle (3) and is attached to a surface (4). Panel A of Figure 1 shows the step of contacting the probe (2) with a sample comprising a target nucleic acid (1). Panel B shows the specific binding of the probe (2) to the target (1). Panel C shows the action of an enzyme (E) which cleaves a hybrid between target and probe nucleic acid. The cleavage of the probe DNA results in the release of the magnetic or magnetizable particle (i.e. the label)(3). Panels D to E show the fate of excess probe which does not bind with DNA target .No hybrid is formed between probe and target (Panel E). Upon action of the enzyme (E) no cleavage occurs. As a result the magnetic or magnetizable particle (i.e. the label)(3) is not released from the surface (Panel F).

During the assay a magnetic force (F) is applied away from the surface such that labels which are freed due to enzyme cleavage are rapidly and continuously removed from the surface.

### Example 2 : Real-time quantitative detection of signal amplification.

The example is illustrated by Figure 2. A probe (2), with a magnetic or magnetizable particle (3) is attached to a surface (4) and is exposed to a sample containing the target (1). Target (1) and probe (2) are complementary and bind specifically to each other. Enzyme (E) can digest probe nucleic acid (2), but not target nucleic acid (1), but only under the condition that the probe it is bound to target. Upon digestion, magnetic or magnetizable particle (i.e. label) (3) and the intact target are released. The intact target can thus hybridize again to another probe (2) present on the reaction surface. This again causes the new probe to be susceptible to digestion, with release of another label (3). As a consequence, the presence of one single target nucleic acid in the sample can cause the release of several labels.

The method of this example can be performed, for example, using an RNA probe and a DNA target with RNAse A as enzyme this enzyme specifically degrades the RNA in RNA:DNA hybrids and will not degrade DNA or unhybridized RNA.

## Claims

1. A method for detecting a target nucleic acid molecule (1) in a sample comprising the steps of:
a) providing a nucleic acid probe (2), comprising a sequence complementary to said target nucleic acid molecule (1), wherein said probe (2) is linked to a magnetic or magnetizable particle (3) and is attached to a surface (4),
b) contacting the sample with the probe under conditions allowing the binding of said probe (2) to said target nucleic acid (1) thereby forming a probe/target hybrid,
c) applying or activating a nucleic acid cleaving enzyme (E), which discriminates between unbound probe and bound probe/target hybrid,
d) applying a magnetic field (F), such as to manipulate unbound magnetic or magnetizable particles towards a zone remote from the surface, at least during step (c),
e) detecting at one or more time points the concentration of magnetic or magnetizable particles on the surface (4) and/or the concentration of magnetic or magnetizable particles in the zone remote from the surface.

2. The method according to claim 1, wherein the detection is performed by measuring a detectable label attached to the magnetic or magnetizable particle.

3. The method according to claim 1, wherein the magnetic or magnetizable particle is a particle comprising magnetic material and a fluorescent label.

4. The method according to claim 1, wherein the nucleic acid cleaving enzyme cleaves at least the probe in the probe/target hybrid.

5. The method according to claim 1, wherein a nicking enzyme is added prior to said nucleic acid cleaving enzyme.

6. The method according to claim 1, wherein the nucleic acid cleaving enzyme is a DNA- or RNAzyme which forms a part of the probe (2).

7. The method according to claim 1, wherein the nucleic acid cleaving enzyme is incorporated in the target nucleic acid molecule (1) during a PCR amplification of the target nucleic acid molecule (1).

8. A device for the detection of magnetic or magnetizable particles comprising:
- a reaction surface (4),
- a first sensor (61) with a first detection zone capable of detecting a label bound to said reaction surface (4),
- a means for applying a magnetic field (F) from the detection surface to a zone remote from the detection surface,
- a second sensor (62) with a second detection zone remote from the detection surface, and
- optionally, a means for moving magnetic or magnetizable particles towards said second detection zone.

9. The device of claim 8, comprising a reaction chamber, wherein said reaction chamber comprises:
- a first region comprising said reaction surface (4) and said first detection zone capable of detecting a label bound to said reaction surface (4),
- a second region remote from the first region, comprising said second detection region capable of detecting a label in said region remote from the first region, and
- a means for applying a magnetic field (F) from the first region to the second region.

10. The device according to claim 9, wherein said first and/or second sensors are capable of detecting different labels.

11. The device according to claim 10, further comprising a plurality of different probes with magnetic or magnetizable particles attached to said reaction surface, wherein each probe comprises a different detectable label.

12. The device according to claim 9, wherein the first and second region are on opposite sides of the reaction chamber.
